# EUROPEAN PATENT SPECIFICATION

(11) **EP 3 630 020 B1**
(45) Date of publication and mention of the grant of the patent: **22.11.2023**
(21) Application number: 18743874.2
(22) Date of filing: 23.05.2018
(51) Int. Cl.: A61F 2/52, A41C 3/10, A41C 3/00

(54) **MEDICAL DEVICE FOR POSTOPERATIVE COMPRESSION FOLLOWING BREAST RECONSTRUCTION SURGERY**
MEDIZINISCHE VORRICHTUNG ZUR POSTOPERATIVEN KOMPRESSION NACH EINER CHIRURGIE ZUR REKONSTRUKTION DER BRUST
DISPOSITIF MEDICAL POUR LA COMPRESSION POST-OPERATOIRE SUIVANT UNE CHIRURGIE DE RECONSTRUCTION DU SEIN

(30) Priority: 24.05.2017 IT 201700056194 U
(43) Date of publication of application: 08.04.2020
(73) Proprietor: DECO Med Srl, 30020 Marcon (VE) (IT)
(72) Inventor: BERTOLI, Giovanni, 30020 Marcon (VE) (IT)
(86) International application number: PCT/IT2018/050089
(87) International publication number: WO 2018/216041

(56) References cited:
- CN-U- 205 094 794
- US-A- 146 805
- US-A1- 2016 015 090
- US-B1- 6 540 702

## Description

This application concerns a medical device for postoperative compression following breast reconstruction surgery after mastectomy for breast cancer. Document CN205094794 U is regarded as the closest prior art.

Today breast surgery is performed for multiple purposes. Nipple sparing, skin sparing and skin reducing mastectomy, quadrantectomy and reconstruction with prostheses or flaps is the surgical treatment for cancer. For immediate reconstruction, it can make use of biological matrices that are used as "complementary fabric" to create the pocket where the prosthesis will be placed.

In non-immediate, two-time reconstructions, an expander is inserted under the greater pectoral muscle, and after it has performed its function, the expander is replaced by the final prosthesis during a second surgical procedure that is performed a few months later.

In aesthetic surgery an additive prosthesis is surgically inserted to increase breast volume. It can be inserted under the gland or under the muscle.

Mastopexy is a breast lifting procedure to control ptosis.

Several scientific studies have recently explored interactions between implanted biomaterials and patient tissues in order to understand the incorporation mechanisms and, especially, to limit postoperative complications, including, in the first place, serum production which,when uncontrolled, is the first step towards surgical failure.

A seroma is accumulation of fluid produced by the body as a result of the inflammatory process induced by surgery.

Within a range of 20-30 cc daily, it does not constitute a danger, but if daily fluid build-up is still around 100 cc 10 days after the implant, this is an alarm sign for the surgeon as it can constitute a risk for the onset of infection.

A seroma can still hinder intimate contact between the biological matrix, when implanted, and vascularised subcutaneous tissue because it is interposed between the two and, therefore, prevents the former's integration.

It can cause dehiscence of the surgical incision with subsequent exposure of the breast implant.

Well known scientific studies have proven that empty spaces inside the mammary chamber encourage serum build up during the postoperative period. Recent innovations in the field of surgical techniques encourage obliteration of dead spaces during surgery by suturing biological matrix and subcutaneous tissues, by using fibrin glue to make the various fasciae adhere together, and by using the biological matrix to form a taught lining for the breast prosthesis.

Even during the postoperative period, compression must be exerted on the breast for at least 15 days to ensure that subcutaneous tissues adhere to the implanted biomaterials to avoid the formation of dead spaces and, hence, serum build-up. The specific case of implantation of a submuscular expander presents the particular need to modulate the external compression gradient based on the variation in expansion of the expander itself, which shifts from 200 cc to ca. 400 cc in a medium breast over a period of six months. It is obvious that, as the internal submuscular expander is gradually inflated, external compression must be reduced to maintain a constant pressure gradient on breast skin.

Today, compression on breast skin is exerted during the postoperative phase through tissues that have a differentiated structure, which can be more or less compressed. These are the so-called containment sports bras or bandage bands, which feature a minimum or almost absent projection of the cup in order to exert frontal compression on the areola-nipple complex. These devices minimise breast movements but, apart from compression of the areola-nipple complex, they do not compress breast skin along an orthogonal axis.

Indeed, today there is no device that compresses certain areas of the breast in a targeted manner, applying less compression on other areas. Recent studies in the field of regenerative surgery and of the tissue repair process reveal how postoperative compression must be performed homogeneously on all breast skin, which is entirely involved in the inflammatory/regenerative process, especially if breast reconstruction was performed using biological matrices that, to become self-tissue, need intimate contact with subcutaneous tissue.

Even during the postoperative phase, this area needs compression to maintain said adherence for at least 15 days.

The only point that can be maintained free of pressure gradients is the areola-nipple complex, since this part of the breast is more susceptible to perfusion problems and, therefore, to a greater risk of cutaneous necrosis.

Frontal compression of the areola-nipple complex is not recommended, but orthogonal compression to the skin surface must be exerted on the four quadrants of the breast to ensure stable intimate contact between subcutaneous tissue and prosthetic implants in order to obliterate dead spaces where serum can build-up inside mammary spaces.

Today certain devices and intimate clothing items possess inflatable structures. But these structures are completely different and are designed for other purposes.

For instance, patent US9468236 claims an intimate garment, a bra (and not a medical device like this invention) with an inflatable structure in each cup, which is more or less spherical, is not divided into quadrants, has no varied pressure gradients, and is positioned in the inferior area of the cup for the sole purpose of making the bra more comfortable. The device is studied to better adapt to the woman's body without absolutely solving the problem of pressure gradient required to obliterate dead spaces.

### SCOPE

The main purpose of this invention is, therefore, to solve the technical issues underscored by eliminating the inconveniences of currently available medical devices.

In fact, the purpose of this invention is to provide compressive and variable orthogonal vectors along the four quadrants of the breast, leaving the central area that corresponds to the areola-nipple complex free. This bra has minimally projecting cups, which internally contain, in the area that interfaces with the skin of the operated breast, several air chambers designed to be inflated with precise Pascal values in order to exert adjustable orthogonal compression on breast skin.

This device ensures obliteration of dead spaces inside the breast chamber between the various implanted materials and vital tissues.

Obliteration of dead spaces diminishes the risk of serum build-up, favouring excellent postoperative recovery.

The invention presents as a bra with two non-preformed cups that are totally closed for 360° without a cleavage between the breasts, and which contain modules that can be inflated according to defined pressure gradients. It is made of non-elastic tissue particularly in the anterior area under the breast groove, and in the subaxillary and posterior area. It also includes two straps that cross at the back and close like a belt on the front.

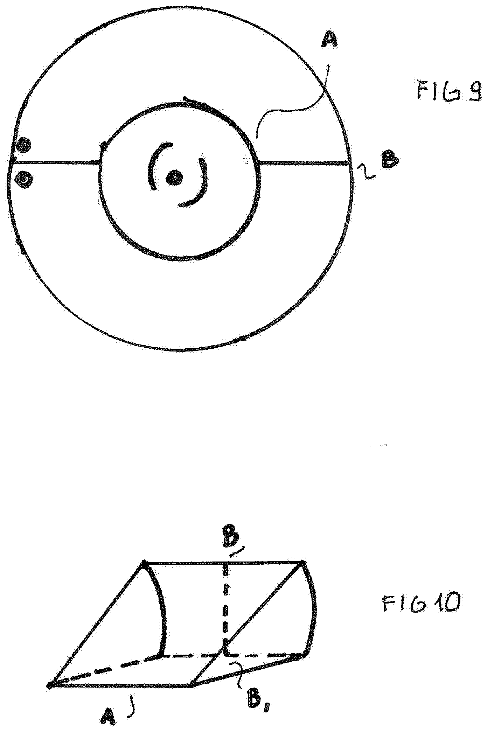

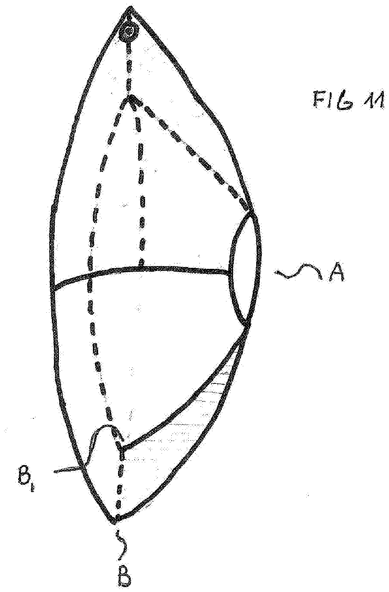

The different pressure gradients are created by introducing various quantities of air, and also by the shape of the inflatable modules. A sagittal section (Fig. 9-10-11) reveals that they are tapered near the areola-nipple complex and are broader towards the chest. Hence lesser pressure can be exerted on the area adjacent to the areola-nipple complex, with enhanced pressure on peripheral breast skin.

The non-elastic tissue prevents the cups from moving away from breast skin when the air chambers are inflated. Hence it ensures that the cups adhere to the breast, allowing air chamber expansion to perform the established axial compression on the breast skin with which it interfaces.

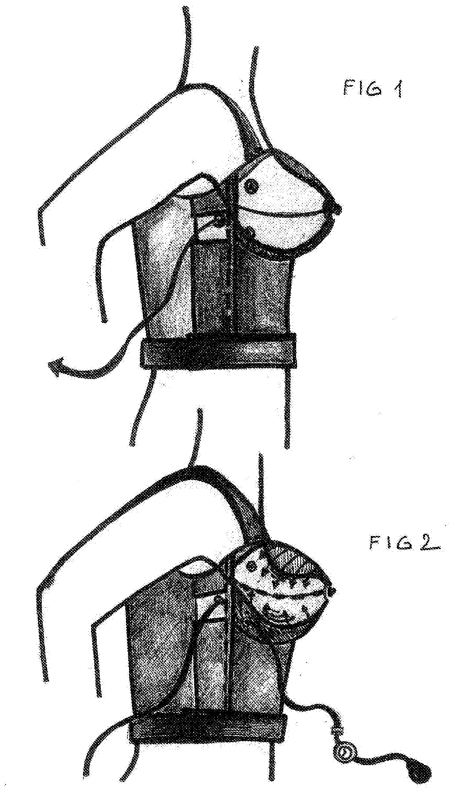

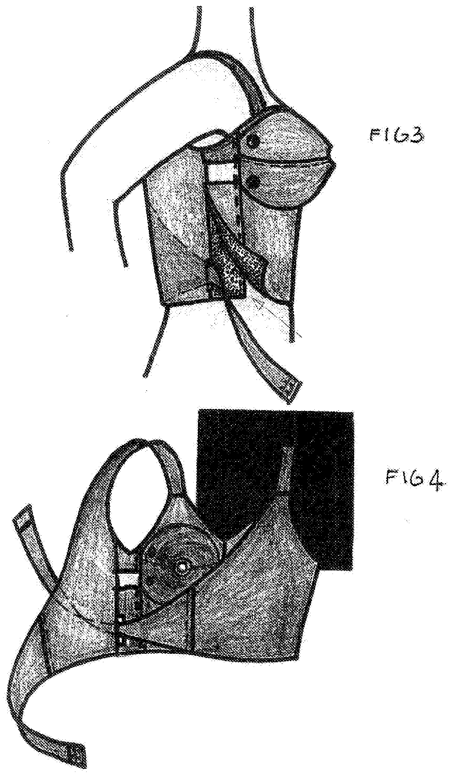

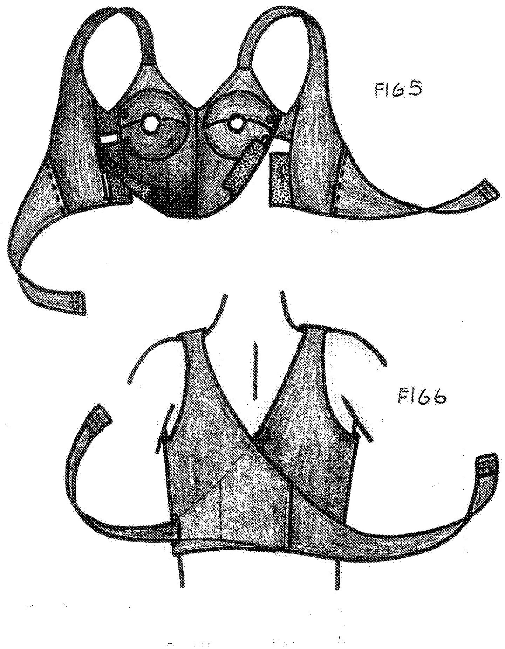

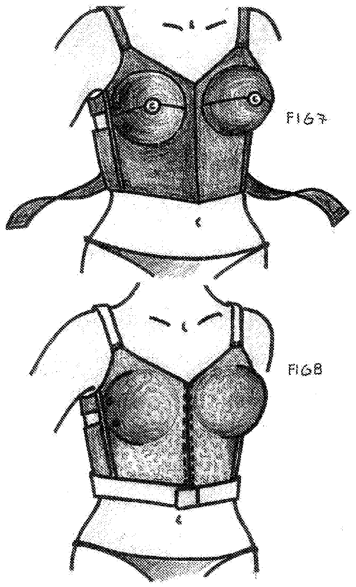

The above straps are made of non-elastic tissue (Fig. 1-2-3-4-5-6-7-8). A Velcro clasp in the subaxillary area allows the bra to be used even with drainage tubes (Fig. 3). The overall item is a band that allows thoracic expansion for breathing but which, at the same time, maintains compression on breast quadrants.

The hollow part of every cup that is in contact with the skin has air chambers in a number ranging from one to a multiple of one, placed in a configuration to spare the areola-nipple complex from pressure, and to involve it when clinically required (e.g., mastopexy or quadrantectomy) (Fig. 1-2).

Air chamber pressure values are defined by the surgeon, depending on the need for compression, and are applied on the various quadrants of the breast, depending on clinical needs, by means of air injected through a manual pump that, along with the pressure gauge, is fitted, from time to time, to the various non-allergic plastic valves in the various quadrants of the breast (Fig. 1-2-3).

As with breast skin, when operated to remove lymph nodes, the armpit too could need compression designed to obliterate empty spaces by means of bringing internal tissues together. The invention, second element of the system, is shaped according to the complex anatomy of shoulder and armpit.

It is made up of an element, a broad and fitting shoulder strap (Fig. 4-5-6), that is connected through an extension of non-elastic tissue towards the armpit, to the underarm that internally contains, in contact with the skin, an inflatable air chamber that exerts an orthogonal pressure gradient on the axillary cavity. This element can be connected to the compressive bra by means of LYCRA fabric ^{®} or VELCRO^{®} to prevent it from slipping laterally.

Additional characteristics and benefits of the invention will be underscored by a detailed description of a specific feature, a non-exclusive form of construction that is illustrated as a rough indication and is not limited to the enclosed drawings in which:
Fig. 1 section of the invention with the air chamber not inflated;
Fig. 2 section of the invention with inflated air chambers;
Fig. 3 lateral view of the invention without tissue lining;
Fig. 4 posterior view of the invention without tissue lining;
Fig. 5 open posterior view of the invention without tissue lining;
Fig. 6 posterior view of the invention, clasped, with tissue lining;
Fig. 7 front view of the invention, without tissue lining;
Fig. 8 front view of the invention without tissue lining;
Fig. 9-10-11 illustrates the inflatable modules (air chambers), front view and section. In A the air chamber has a height (thickness) of zero, which is constant along the entire circumference. This height (thickness) increases proportionately as B draws close, and the air chamber has a constant height (thickness) B-B1 along the entire circumference.

It must be said that all information already known during the patent application procedure is intended as not claimed and the subject of disclaimer. In any case, the materials used and the dimensions of the invention can be more relevant depending on the specific needs.

The various means to perform certain different functions must not coexist only in the form of product described, but can be present in many forms of product that are even not illustrated.

The characteristics indicated as benefits, appropriate and so one can also be removed.

## Claims

1. A medical device for the postoperative compression of the skin after breast reconstruction providing compressive and variable orthogonal vectors along the four quadrants of the breast and leaving the areola-nipple complex tree said medical device comprising a female breast support garment, with two cups containing several inflatable modules designed to be inflated in order to exert adjustable ortnogonai compression on breast sKin, **characterized in that** said inflatable modules are tubular and define a trunked cone shape with variable thickness from the top (A) to the bottom (B) in order to generate a pressure gradient reaching orthogonal, compressive and different vectors on the four quadrants of the breast and leaving empty the areolar nipple complex.

2. A medical device as claimed in claim 1, **characterized in that** the inflatable tubular modules have a minimum thickness at the nearest part (A) of the areolar nipple complex and maximum thickness at the far end (B) of the areolar nipple complex.

3. A medical device as claimed in claims 1 and 2, **characterized in that** the inflatable modules are 2 or multiple of 2 and are inflatable separately through various valves.

4. A medical device as claimed in claims 1, 2 and 3, **characterized in that** it is constituted by inelastic material.

5. A medical device as claimed in claims 1, 2, 3 and 4, **characterized by** having two inelastic straps that intersect on the back and then close like a waistband in the front.

## Patentansprüche

1. Ein Medizinprodukt für die postoperative Hautkompression nach einer Brustrekonstruktion mit komprimierenden und variablen orthogonalen Trägern entlang der vier Quadranten der Brust, wobei der Mamillen-Areolen-Komplex frei bleibt; es besteht in einem BH mit zwei Körbchen, die mehrere Module enthalten, die so aufgebaut sind, dass sie unterschiedlich stark aufgeblasen werden können, um eine individuell einstellbare orthogonale Kompression auf die Brusthaut auszuüben. Das Produkt ist durch eine schlauchartige Ausführung der aufblasbaren Module gekennzeichnet, die einen Kegelstumpf bilden, dessen Dicke von oben (A) nach unten (B) individuell verändert werden kann, um einen an jedem der vier Quadranten der Brust unterschiedlichen orthogonalen Druckgradienten erzeugen zu können und den Mamillen-Areolen-Komplex freizulassen.

2. Ein Medizinprodukt entsprechend Anspruch 1, das **dadurch gekennzeichnet ist, dass** die aufblasbaren Schlauchmodule am dem Mamillen-Areolen-Komplex nächstgelegenen Abschnitt (A) die geringste Dicke und am dem Mamillen-Areolen-Komplex fern gelegenen Ende (B) die größte Dicke aufweisen.

3. Ein Medizinprodukt entsprechend den Ansprüchen 1 und 2, das **dadurch gekennzeichnet ist, dass** die Anzahl der unabhängig voneinander durch verschiedene Ventile aufblasbaren Module 2 oder ein Vielfaches von 2 ist.

4. Ein Medizinprodukt entsprechend den Ansprüchen 1, 2 und 3, das **dadurch gekennzeichnet ist, dass** es aus einem nicht elastischen Material besteht

5. Ein Medizinprodukt entsprechend den Ansprüchen 1, 2, 3, 4, das **dadurch gekennzeichnet ist, dass** es zwei nicht elastische Riemen aufweist, die sich auf dem Rücken kreuzen und dann wie ein Taillenband vorne geschlossen werden.

## Revendications

1. Dispositif médical pour la compression postopératoire de la peau après une reconstruction mammaire, fournissant des vecteurs orthogonaux compressifs et variables le long des quatre quadrants du sein et laissant libre le complexe aréole-mamelon, ledit dispositif médical comprenant un sous-vêtement de soutien du sein de femme, avec deux bonnets contenant plusieurs modules gonflables conçus pour être gonflés afin d'exercer une compression orthogonale réglable sur la peau du sein, **caractérisé par le fait que** les modules gonflables sont tubulaires et définissent un tronc de cône d'épaisseur variable de la partie supérieure (A) à la partie inférieure (B) afin de générer un gradient de pression atteignant des vecteurs orthogonaux, compressifs et différents sur les quatre quadrants du sein et laissant vide le complexe aréolo-mamelonnaire.

2. Dispositif médical selon la revendication 1, **caractérisé par le fait que** les modules tubulaires gonflables ont une épaisseur minimale au niveau de la partie la plus proche (A) du complexe aréolo-mamelonnaire et une épaisseur maximale au niveau de l'extrémité la plus éloignée (B) du complexe aréolo-mamelonnaire.

3. Dispositif médical selon les revendications 1 et 2, **caractérisé par le fait que** les modules gonflables sont au nombre de 2 ou d'un multiple de 2 et qu'ils sont gonflables séparément par l'intermédiaire de diverses valves.

4. Dispositif médical selon les revendications 1, 2 et 3 **caractérisé par le fait qu'**il est constitué d'un matériau inélastique

5. Dispositif médical selon les revendications 1, 2, 3, 4, **caractérisé par** la présence de deux bretelles inélastiques qui se croisent dans le dos et se ferment ensuite comme une ceinture à l'avant.
